# EUROPEAN PATENT SPECIFICATION

(11) **EP 0 509 040 B1**
(45) Date of publication and mention of the grant of the patent: **09.11.1994**
(21) Application number: 91902626.0
(22) Date of filing: 04.01.1991
(51) Int. Cl.: A61K 37/43, A61K 37/02, A61K 37/36, A61K 37/38

(54) **METHOD FOR INCREASING FERTILITY IN FEMALES**
METHODE ZUR ERHÖHUNG DER FRUCHTBARKEIT WEIBLICHER SÄUGER
PROCEDE DESTINE A ACCROITRE LA FERTILITE FEMININE

(30) Priority: 08.01.1990 US 461713
(43) Date of publication of application: 21.10.1992
(73) Proprietor: GENENTECH, INC., South San Francisco, CA 94080-4990 (US)
(72) Inventor: WOODRUFF, Teresa, K., Millbrae, CA 94030 (US); MATHER, Jennie, P., Millbrae, CA 94030 (US)
(74) Representative: Nicholls, Kathryn Margaret
(86) International application number: US9100079
(87) International publication number: WO9110445

(56) References cited:
- WO-A-86/00078
- WO-A-88/00208
- US-A- 4 737 578
- Journal 0f endocrinology, vol., 102, 1984, K. M. Henderson et al., Increase in ovulation rate after active immunization of sheep with inhibin partiallypurifed from bovine follicular fluid", pages 305-309,.
- Physiological reviews, vol., 68, no. 2, April 1988, F. H. De Jong, "Inhibitin",pages 588-593, see pages 592-593, VIII. Summary and conclusions.

## Description

### Background of the Invention

### Field of the Invention

This invention relates to the use of inhibin to increase fertility in female mammals.

### Description of Related Art

Inhibin is a glycoprotein produced by diverse tissues, including the gonads, pituitary, brain, bone marrow, placenta, and adrenal gland. It was initially identified by its ability to inhibit the secretion of follicle stimulating hormone (FSH) by the pituitary. De Jong and Sharpe, Nature, 263: 71-72 (1976); Schwartz and Channing, Proc. Natl. Acad. Sci. USA, 74: 5721-5724 (1977). Such preferential regulation of the gonadotropin secretion has generated a great deal of interest and prompted many laboratories in the past fifty years to attempt to isolate and characterize this substance from extracts of testis, spermatozoa, rete tests fluid, seminal plasma, and ovarian follicular fluid using various bioassays. River *et al.,* Biochem. Biophys. Res. Commun., 133: 120 (1985); Ling *et al.,* Proc. Natl, Acad. Sci. USA, 82: 7217 (1985); Fukuda et al., Mol. Cell Endocrinol., 44: 55 (1985). The structure of inhibin, characterized from several species, consists of two disulfide-linked subunits; an α and either a, βA or a βB chain.

After the identification of inhibin, activin was shown to exist in follicular fluid as a naturally occurring substance. Activin was found to be capable of stimulating FSH release by rat anterior pituitary cells. Vale *et al*., Nature, 321: 776-779 (1986); Ling *et al*., Nature, 321: 779-782 (1986). Activin consists of a homodimer or heterodimer of inhibin β subunits, which may be β_{A} or β_{B} subunits. Vale *et al*., Recent Prog. Horm. Res., 44: 1-34 (1988). There is 95-100% amino acid conservation of β subunits among human, porcine, bovine, and rat activins. The β_{A} and β_{B} subunits within a given species are about 64-70% homologous. The activin β_{A} and β_{B} homodimers ("Activin A" and "Activin B," respectively) have been identified in follicular fluid, and both molecules have been cloned and their genes expressed. Mason *et al*., Biochem. Biophys. Res. Commun., 135: 957 (1986); EP Pub. No. 222,491 published May 20, 1987; Mason *et al*., Molecular Endocrinol., 3: 1352-1358 (1989). The complete sequence of the β_{B} subunit is published in Serono Symposium Publications, entitled "Inhibin- Non-Steroidal Regulation of Follicle Stimulating Hormone Secretion", eds. H.G. Burger *et al*., abstract by A.J. Mason *et al*., vol. 42, pp. 77-88 (Raven Press, 1987), entitled "Human Inhibin and Activin: Structure and Recombinant Expression in Mammalian Cells."

Both Activin A and Activin AB, but thus far not Activin B, have been isolated from natural sources. Activin mRNA (β_{A} and β_{B} subunits), bioactivity, and immunoactivity have been reported to be produced by testicular Leydig cells from immature rat and pig. Lee *et al*., Science, 243: 396-398 (1989); Lee *et al*., in Serono Symposium Publications, entitled "The Molecular and Cellular Endocrinology of the Testis" eds. Cooke and Sharpe, Vol. 50 (Raven Press: New York, 1988), p. 21-27. Activin A has been found recently to have erythropoietic-stimulating activity as well as FSH-releasing activity. See EP Publ. No. 210,461 published February 4, 1987 (where the protein is called BUF-3), Eto *et al*, Biochem. Biophys. Res. Commun., 142: 1095-1103 (1987) and Murata *et al*., Proc. Natl. Acad. Sci. U.S.A., 85: 2434-2438 (1988) (where the activin is called EDF), and Yu *et al*., Nature, 330: 765-767 (1987) (where the activin is called FRP). In these systems, inhibin antagonized the actions of activin.

Recently, the expression of inhibin subunits, each encoded by a separate gene, was demonstrated in several tissues in addition to ovary and testis. Inhibin α, β_{A}, and β_{B} mRNAs were detected in placental, pituitary, adrenal, bone marrow, and brain tissues. Meunier *et al*., Proc. Natl. Acad. Sci. USA, 85: 247-251 (1988). The expression of the inhibin subunit mRNAs varied by several-fold in a tissue-specific manner, suggesting different functions for these proteins depending on their pattern of association and their site of production.

Inhibin and activin are members of a family of growth and differentiation factors. The prototype of this family is transforming growth factor-beta (TGF-β) (Derynck *et al*., Nature, 316: 701-705 (1985)), which, according to one source, also possesses FSH-releasing activity. Ying *et al*., Biochem. Biophys. Res. Commun., 135: 950-956 (1986). Other members of the TGF-β family include the Mullerian inhibitory substance, the fly decapentaplegic gene complex, and the product of Xenopus Vg-1 mRNA.

In the human, growing preovulatory follicles and corpus luteum secrete inhibin into the circulation in response to FSH stimulation. Lee and Gibson, Aust. J. Biol. Sci., 38: 115-120 (1985); McLachlan *et al*., Fertil. Steril., 48: 1001 (1987). Thus, inhibin-related peptides play important roles in the modulation of gonadal functions via a pituitary feedback loop. In rat primary cultures of tests cells and ovarian thecal-interstitial cells, inhibin has been reported to enhance androgen biosynthesis stimulated by leutinizing hormone (LH), whereas activin suppresses androgen production. Hsueh *et al*., Proc. Natl. Acad. Sci. USA, 84: 5082-5086 (1987). Other workers have been unable to repeat these observations. deKretser and Robertson, Biology of Reproduction, 40: 33-47 (1989).

Inhibitory effects of TGF-β on Leydig cell steroidogenesis have also been described. Lin *et al*., Biochem. Biophys. Res. Commun., 146: 387 (1987); Fauser and Hsueh, Life Sci., 43: 1363 (1988); Avallet *et al*., Biochem. Biophys. Res. Commun., 146: 575 (1987). In granulosa cells, activin has been reported to inhibit (and TGF-β to enhance) progesterone production. Ignotz and Massague, J. Biol. Chem.., 261: 4337 (1986). In primary cultures of granulosa cells, activin and inhibin as well as TGF-β were found to affect hormone synthesis and secretion, each in a different fashion. Adashi and Resnick, Endocrinology, 119: 1879 (1986); Ying *et al*., Biochem. Biophys. Res. Commun., 136: 969 (1986); Hutchinson *et al*., Biochem. Biophys. Res. Commun., 146: 1405 (1987); Mondschein *et al*., Endocrinology, 123: 1970 (1988); Feng *et al*., J. Biol. Chem., 261: 14167 (1986). These molecules have both positive and negative effects on FSH-dependent granulosa cell function. Carson *et al*., J. Reprod. Fert., 85: 735-746 (1989). Also suggested is that individual members of the TGF-β/inhibin gene family regulate ovarian function, not only by direct action on follicle cells, but also indirectly by influencing the production rate of other members of that family. Zhiwen *et al*., Molecular and Cellular Endocrinology, 58: 161-166 (1988).

Activin A and inhibin were reported to modulate growth of two gonadal cell lines, suggesting that these proteins may regulate proliferation as well as functions of gonadal cells. Gonzalez-Manchon and Vale, Endocrinology, 125: 1666-1672 (1989). The secretion of inhibin by the corpus luteum has been proposed to suppress the concentration of FSH in the luteal phase of the cycle and hence the inhibition of follicular development. Baird *et al*., Ann. N. Y. Acad. Sci., 541: 153-161 (1988).

A review article postulates that inhibin is at least one of the factors that determines the number of follicles destined to ovulate, and that interference with the action of inhibin might contribute to the regulation of fertility. De Jong, Physiol. Rev, 68: 555 (1988). Many investigators have speculated that due to its FSH-inhibiting effect at the level of the pituitary, inhibin may be useful in male and female contraception. Sheth and Moodbidri, Adv. Contracept. 2: 131-139 (1986); Findlay, Fertil. Steril., 46: 770 (1986). Another author doubts that inhibin can inhibit spermatogenesis (citing Bremner *et al*., J. Clin. Invest., 68: 1044 (1981)), and states that inhibin might also have some direct stimulatory effects on spermatogenesis. Baker *et al*., Clin. Reprod. and Fert., 2: 161-174 (1983).

When sheep are immunized with inhibin or the inhibin α chain, their ovulation rate is increased, due to the immunoneutralization of endogenous inhibin. Cummins *et al*., J. Reprod. Fertil., 77: 365 (1986); Henderson *et al*., J. Endocrinol., 102: 305-309 (1984); Forage *et al*., J. Endocrinol., 114: R1 (1987); Al-Obaidi *et al*., J. Reprod. Fert., 81: 403-414 (1987). The same effect has been observed in rats. River and Vale, Endocrinology, 125: 152 (1989). In addition, Rivier and Vale suggest that increased FSH alone is sufficient to stimulate additional follicular growth and development, and the main mechanism through which treatment with anti-inhibin serum increases follicular development is through elevated plasma FSH levels. Other investigators reported that the administration of inhibin to sheep induces either anovulation or an increase in ovulation rate according to the scheme of treatment. Franchimont *et al*., Rev. fr. Gynecol. Obstet., 83: 607 (1988).

The modulation of the inhibin subunit mRNAs during the rat estrus cycle has been intensely studied. Woodruff *et al*., Science, 239: 1296 (1988). Only recently has the integrative feedback relationship between ovarian inhibin and activin and pituitary FSH been partially elucidated. Hasegawa *et al*., in Inhibin: Non-Steroidal Regulation of FSH Secretion, ed. J. Burger *et al*., 42: 119-133 (New York: Raven Press, 1987); Woodruff *et al*., Science, 239: 1296-1299 (1988). Briefly, the ovary produces low levels of inhibin on the evening of proestrus. This allows FSH to remain elevated throughout the morning of estrus (secondary FSH surge). Rivier *et al*., Science, 234: 205-208 (1986). The secondary FSH surge recruits a new set of follicles into the ovulatory pool and is responsible for the initiation of inhibin subunit mRNA expression. As a consequence of inhibin production, pituitary FSH secretion is downregulated. Inhibin mRNA levels increase in maturing follicles as they progress through the cycle. Follicles that become atretic (non-ovulatory and highly steroidogenic) have little or no inhibin mRNA. Woodruff *et al*., in Growth Factors and the Ovary, ed. Hirshfield, pp. 291-295 (New York: Plenum Press, 1989). Inhibin subunit mRNA accumulation climaxes on the afternoon of proestrus in healthy follicles simultaneously with the primary LH and FSH surges. Woodruff *et al*., Science, *supra.*

Since the first successful *in vitro* fertilization procedure in 1978, attempts have been made to employ drugs or hormones to induce multiple follicular and oocyte development. Thus, *in vitro* fertilization pregnancies could result in women following the use of clomiphene citrate and human chorionic gonadotrophin to induce multiple follicular development in endocrine normal patients. Trounson *et al*., Science, 212: 681-684 (1981). It is well established that the appropriate application of mixed exogenous gonadotropins FSH and LH has proven efficacious for ovulation induction or for multiple egg retrieval during *in vitro* fertilization therapy. However, ovarian stimulation by administration of exogenous gonadotropins is difficult to manage. Another agent of some use is FSH alone or in combination with a gonadotropin releasing hormone antagonist, as described in U.S. Pat. No. 4,845,077.

The treatments with FSH or a mixture of FSH and LH result in increased inhibin serum levels during the follicular and early luteal phase of the cycle. McLachlan *et al*., Lancet, 1: 1233-1234 (1986); Tsonis *et al*., J. Clin. Endocrinol. Metab., 66; 915 (1988); Buckler *et al*., J. Endocrin., 122: 279-285 (1989); Tsuchiya *et al*., Fert. Steril., 52: 88 (1989). However, another investigator has found that at the time of ovulation neither inhibin activity nor follicular levels of steroid or gonadotropins are adequate criteria for predicting the eventual outcome of oocytes in an *in vitro* fertilization protocol. Lefevre *et al*., Fert. Steril., 46: 325 (1986).

It is an object of this invention to employ inhibin to increase fertility in those patients that do not respond to gonadotropin or other treatments.

It is another object to induce ovulation for the purpose of *in vitro* fertilization and embryo transfer.

These and other objects will be apparent to one of ordinary skill in the art.

### Summary of the Invention

A method of increasing fertility in a female mammal comprising administering to the ovary of the mammal an effective amount of inhibin.

In another aspect, the invention provides a pharmaceutical composition for increasing fertility comprising an effective amount of inhibin in a pharmaceutically acceptable carrier.

Inhibin has been found to be a regulator of follicular development during the transition from diestrus to proestrus. *In vivo* studies were conducted by unilateral injection of inhibin into immature female rat ovaries and analysis of maturation of the antral follicle by morphology, follicular diameter, and incorporation of 3H-thymidine into developing follicles. The results indicate surprisingly that growth and development of the ovarian follicle can be maintained by local administration of inhibin to the ovary. Thus, females with infertility due to inadequate follicular development unresponsive to gonadotropin treatment have available an alterative therapy.

### Brief Description of the Drawings

Figure 1 is a graph of the follicular distribution in control (saline) and pregnant mare serum gonadotropin (PMSG)-treated ovaries of rats.

Figure 2 is a graph of the follicular distribution in inhibin-and activin-treated ovaries, with the asterisks indicating largely atretic responses.

### Description of the Preferred Embodiments

As used herein, the term "inhibin" refers to the heterodimers of α and β chains of inhibin, prepro forms, and pro forms, together with glycosylation and/or amino acid sequence variants thereof. After cleavage from the mature protein, the precursor portion may be non-covalently associated with the mature protein. Inhibin A refers to inhibin with the chains α and β_{A}. Inhibin B refers to inhibin with the chains of α and β_{B}.

The intact isolated prepro or prodomain or mature β_{A}, β_{B}, and α sequences are suitably synthesized by any means, including synthetic and/or recombinant means, but are preferably synthesized in recombinant cell culture, for example, as described in U.S. Pat. No. 4,798,885 issued January 17, 1989.

It is within the scope hereof to employ inhibin from animals other than humans, for example, porcine or bovine sources, to treat humans. For example, the nucleotide and deduced amino acid sequences of the porcine inhibin α chain are found in U.S. Pat. No. 4,798,885, *supra.* Likewise, if it is desirable to treat other mammalian species such as domestic and farm animals and zoo, sports or pet animals, human inhibin, as well as inhibin from other species, is suitably employed.

Generally, amino acid sequence variants will be substantially homologous with the relevant portion of the mammalian α or β chain sequences set forth in, e.g., U.S. Pat. No. 4,798,885, *supra.* Substantially homologous means that greater than about 60% of the primary amino acid sequence of the homologous polypeptide corresponds to the sequence of the inhibin chain when aligned to maximize the number of amino acid residue matches between the two proteins. Alignment to maximize matches of residues includes shifting the amino and/or carboxyl terminus, introducing gaps as required, and/or deleting residues present as inserts in the candidate. Typically, amino acid sequence variants will be greater than about 70% homologous with the corresponding native sequences.

While the site for introducing a sequence variation is predetermined, it is unnecessary that the mutation *per se* be predetermined. For example, in order to optimize the performance of mutation at a given site, random mutagenesis may be conducted at the target codon or region and the expressed inhibin mutants screened for the optimal combination of desired activity. Techniques for making substitution mutations at predetermined sites in DNA having a known sequence are well known, for example, M13 primer mutagenesis.

Mutagenesis is conducted by making amino acid insertions, usually on the order of about from 1 to 10 amino acid residues, or deletions of about from 1 to 30 residues. Substitutions, deletions, insertions, or any subcombination may be combined to arrive at a final construct. Preferably, however, substitution mutagenesis is conducted. Obviously, the mutations in the encoding DNA must not place the sequence out of reading frame and preferably will not create complementary regions that could produce secondary mRNA structure.

Covalent modifications of inhibin are included within the scope of the invention, and include covalent or aggregative conjugates with other chemical moieties. Covalent derivatives are prepared by linkage of functionalities to groups that are found in the inhibin amino acid side chains or at the N- or C-termini, by means known in the art. For example, these derivatives will include: aliphatic esters or amides of the carboxyl terminus or residues containing carboxyl side chains, e.g., aspartyl residues; O-acyl derivatives of hydroxyl group-containing residues such as aryl or alanyl; and N-acyl derivatives of the amino terminal amino acid or amino-group containing residues, e.g., lysine or arginine. The acyl group is selected from the group of alkyl moieties (including C3 to C10 normal alkyl), thereby forming alkanoyl species, and carbocyclic or heterocyclic compounds, thereby forming aroyl species. The reactive groups preferably are difunctional compounds known *per se* for use in crosslinking proteins to insoluble matrices through reactive side groups, e.g., m-maleimidobenzoyl-N-hydroxy succinimide ester. Preferred derivatization sites are at histidine residues.

The expression "administering to the ovary" means not only injection into the ovary as by intrabursal means, but also techniques that result in flooding the area surrounding the ovary with inhibin such that the inhibin is absorbed into the ovary. This may be accomplished by making an incision in the abdomen or through the ovary with or without the use of a scope. In addition, the inhibin can be injected into a vessel that feeds the ovary, preferably using a microscopic procedure. Furthermore, the inhibin can be put into an implant that is placed near the ovary and through which the inhibin is absorbed into the ovary. Other techniques may be employed, provided that the result is that inhibin is applied locally to the ovary and is effective for the purposes stated herein.

The present invention concerns itself with using inhibin to increase fertility in female mammals, including sports, zoo, pet, and farm animals such as dogs, cats, cattle, pigs, horses, monkeys, and sheep, as well as humans. The need for an increase in fertility is generally due to inadequate ovarian follicular development.

The inhibin is administered to the mammal by means of the ovary, as discussed above. The specific route of administration will depend, e.g., on the medical history of the patient.

The inhibin compositions to be used in the therapy will be formulated and dosed in a fashion consistent with good medical practice, taking into account the clinical condition of the individual patient, the method of administration, the scheduling of administration, and other factors known to practitioners. The "effective amount" for purposes herein is thus determined by such considerations.

As a general proposition, the total pharmaceutically effective amount of the inhibin administered per dose will be in the range of about 1 µg/kg/day to 10 mg/kg/day of patient body weight, although, as noted above, this will be subject to a great deal of therapeutic discretion. Preferably, this dose is at no more than about 10 µg/kg/day. The key factor in selecting an appropriate dose is the result obtained, as measured by increases in follicular development or by other criteria as deemed appropriate by the practitioner.

For administration, the inhibin is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation. For example, the formulation preferably does not include oxidizing agents and other compounds that are known to be deleterious to polypeptides.

Generally, the formulations are prepared by contacting the inhibin uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes.

The carrier suitably contains minor amounts of additives such as substances that enhance isotonicity and chemical stability. Such materials are non-toxic to recipients at the dosages and concentrations employed, and include buffers such as phosphate, nitrate, and other organic acid salts; antioxidants such as ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine,; monosaccharides, disaccharides, and other carbohydrates including cellulose or its derivatives, glucose, mannose, or dextrins; chelating agents such as EDTA; sugar alcohols such as mannitol or sorbitol; counterions such as sodium, and/or nonionic surfactants such as Tween, Pluronics, or PEG.

The inhibin is typically formulated in such vehicles at a concentration of about 0.1 mg/ml to 100 mg/ml at physiological pH. It will be understood that use of certain of the foregoing excipients, carriers, or stabilizers will result in the formation of inhibin salts.

Inhibin to be used for therapeutic administration must be sterile. Sterility is readily accomplished by filtration through sterile filtration membranes (e.g., 0.2 micron membranes).

Therapeutic inhibin compositions generally are placed into a container having a sterile access port, for example, a vial having a stopper pierceable by a hypodermic injection needle.

Inhibin ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous inhibin solution, and the resulting mixture is lyophilized. The infusion solution is prepared by reconstituting the lyophilized inhibin using 5 ml of sterile water or Ringer's solution.

Inhibin therapy is suitably combined with other proposed or conventional fertility increasing therapies. For example, inhibin can be administered with other fertility agents used to increase the number of ova available. Further, the inhibin can be combined with agents that enhance the effects of or synergize with the known fertility agents such as gonadotropin hormone releasing antagonists.

Examples of agents used to induce controlled multiple follicular maturation include clomiphene citrate or human menopausal gonadotropins, e.g., FSH as described in U.S. Pat. 4,845,077 or a mixture of FSH and LH, and/or human chorionic gonadotropins. To decrease the marked individual variability in response to human menopausal gonadotropin therapy, a gonadotropin releasing hormone antagonist may be administered. Typical gonadotropin releasing hormone antagonists are described by Rees *et al*., J. Med. Chem., 17: 1016 (1974); Coy *et al*., Peptides, 1976 (Loffed Ed., Editions de L'Universite de Bruxelle 1977) p. 463, Beattie *et al*., J. Med. Chem., 18: 1247 (1975); Channabasavaiah *et al*., Biochem. Biophys. Res. Commun., 86: 1266 (1979), and U.S. Pat. Nos. 4,317,815 and 4,431,635. These include (Ac-pClPhe¹, pClPhe², DTrp³, DArg⁶, DAla¹⁰)GnRH HCl, [D-Phe²]-LHRH, [D-Phe², D-Phe⁶]-LHRH, [D-Phe², Phe³, D-Phe⁶]-LHRH, [D-Phe², D-Trp³, D-Phe⁶]-LHRH, [D-p-F-Phe-D-Ala⁶]-LHRH, and [Ac-D-Phe¹, D-Phe², D-Trp^{3,6}]-LHRH.

The inhibin and fertility agents are suitably delivered by separate or the same means, by separate or the same administration route, and at the same or at different times, depending, e.g., on dosing, the clinical condition of the patient, etc. It is not necessary that such fertility agents be included in the inhibin compositions *per se* although this will be convenient where such drugs are delivered by the same administration route.

When employed together with the inhibin, such agents typically are employed in lesser dosages than when used alone. If FSH is used separately, preferably the effective amount is a daily amount of about 70 to 220/I.U., more preferably 1.5 to 4.0 I.U./kg. If a gonadotropin releasing hormone antagonist is used in conjunction with FSH, preferably the amount is and the daily amount of gonadotropin releasing hormone antagonist is about 1.0 to 4.0 mg/kg, more preferably 1.5 to 2.5 mg/kg. Further details on administration of these latter agents can be found in U.S. Pat. No. 4,845,077.

A typical combined composition will contain the above-noted amount of inhibin and from about 70 to 220/I.U. of FSH in a suitable intraperitoneal fluid such as lactated Ringer's solution.

For women undergoing ovulation induction to effect an embryo transfer, any embryo transfer procedure may be employed after ovulation is induced by the inhibin. These include, e.g., *in vitro* fertilization and embryo transfer (IVF-ET) (Quigly *et al*., Fertil. Steril.. 38: 678 (1982)), gamete intrafallopian transfer (GIFT) (Molloy *et al*., Fertil. Steril, 47: 289 (1987)), and pronuclear stage tubal transfer (PROST) (Yovich *et al*., Fertil. Steril, 48: 851 (1987)). Successful such procedures are positively correlated with the number of oocytes retrieved and the number of viable embryos transferred.

When the schedule of ovarian events progresses as expected, a burgeoning follicle(s) on the ovarian surface can be viewed near midcycle by ultrasound or laparoscopy, having distended vessels and substantial translucence. This is the familiar appearance of the dominant follicle near ovulation. For the typical *in vitro* fertilization protocol, a needle is passed into the follicle and its contents, which may be a single oocyte, are aspirated in the spontaneous ovarian cycle. Oocyte recovery is performed by means of transvaginal ultrasonically guided follicular aspiration. Following evacuation, the follicle collapses. When the follicle is aspirated, the ovum is examined microscopically to assess its condition.

Subjective criteria to estimate the normality of the ovum include assessing its viability and ripeness by the number of surrounding granulosa cells, the density of the mucus coating, the thickness of the zona pellucida, and the presence or absence of the polar body. Human tubal fluid, as obtained from Quinn *et al*., Fertil. Steril., 44: 493 (1985), supplemented with 10% heat-inactivated maternal or fetal cord serum, may be used for IVF and embryo culture. Mature oocytes are inseminated with washed and migrated spermatozoa (typically 100,000 to 200,000 per egg) at a determined lapsed time after aspiration, such as 6 to 8 hours. Fertilization is assessed typically 12 to 18 hours after insemination and the oocytes are transferred to growth media. Only normally fertilized oocytes are transferred to the patients at the 2- to 8-cell stage at typically 48 to 56 hours after retrieval. Successful IVF results in pregnancies following transfer of donated embryos.

General protocols for IVF include those disclosed by Trounson *et al*., *supra;* Trounson and Leeton, in Edwards and Purdy, eds., Human Conception*in vitro* (New York: Academic Press, 1982), and Trounson, in Crosignani and Rubin, eds.*, in vitro* Fertilization and Embryo Transfer, p. 315 (New York: Academic Press, 1983).

The threat of non-hormone-induced luteal phase hormonal deficiency that may occur in IVF may be ameliorated by administration of the collective secretions for more than one corpus luteum.

For the GIFT protocol, oocyte recovery is suitably carried out by a standard laparoscopically guided follicular aspiration technique. Renou *et al*., Fertil. Steril, 35: 409 (1981). For a typical procedure, once oocytes are collected and graded, the gametes, a maximum of five oocytes, are transferred to the fallopian tube. A semen sample is collected about 3 to 4 hours before the oocyte collection, and the final suspension of motile spermatozoa is adjusted to 100,000 to 200,000/35 µl. The GIFT catheter is loaded with sperm suspension and oocytes. The fallopian tube is catheterized as described by Molloy *et al*., Fert. Steril., 47: 289 (1987), and the gametes are transferred.

For the PROST protocol, all procedures for oocyte aspiration, semen suspension preparation, and insemination are performed using the same procedure as in the IVF program. After the assessment of oocyte fertilization, two pronuclei oocytes are transferred into the fallopian tube by the same procedure as in the GIFT program.

The invention will be more fully understood by reference to the following examples. They should not, however, be construed as limiting the scope of the invention.

### EXAMPLE I

Twenty-five-day-old female Sprague-Dawley rats (Charles River Laboratories, Inc., Wilmington, MA) were anesthetized with a combination of 80 mg/kg of ketamine HCl (Vetlalar, Aveco Company) and 4 mg/kg xylozine (Gemini, Rugby Laboratories). The left dorsal lumbar region of the rat was then clipped and prepared with 70% isopropyl alcohol and an iodine scrub solution. A 5-7 mm incision was made through the dermal layers, just caudal to the last rib, followed by a 3-5-mm cut into the peritoneum. The ovary was then visualized and gently exteriorized by grasping the surrounding fat. Placing a pair of straight-eye dressing forceps between the left horn of the uterus and the surrounding fat and allowing them to open presented the ovary for injection. While the attached fat was grasped for stabilization, 10 µl of human recombinant inhibin A or activin A (prepared and purified as described in U.S. Pat. No. 4,798,885 issued January 17, 1989) was injected (1 µg/ovary) intrabursally caudal to the oviduct through a 28-gauge needle attached to a tuberculin syringe. PMSG, which is a combination of FSH and LH, was injected intraperitoneally at 25 international units (IU).

Animals were divided into the following groups:
Group 1: saline (n=5);
Group 2: PMSG (n=6);
Group 3: inhibin A (n=8);
Group 4: inhibin A and PMSG (n=7);
Group 5: activin A (n=7);
Group 6: activin A and PMSG (n=8).
The total number of animals used in each experiment is given in parenthesis; this represents two independent repetitions. 3H-Thymidine (1 µCi) was coinjected intrabursally with the hormones indicated below in an additional set of animals:
Group 7: 3H-thymidine in saline;
Group 8: 3H-thymidine and PMSG;
Group 9: 3H-thymidine and inhibin A;
Group 10: 3H-thymidine, inhibin A, and PMSG.

For each animal one ovary was injected with hormone and the other ovary served as the contralateral control. Dose volume was approximately 10 µl. Immediately post-dosing, slight pressure was applied to the injection site with a cotton applicator stick. The ovary was then carefully replaced, the incision was closed with two 9-mm wound clips, and the animal was returned to its cage and observed until it had recovered its righting reflex.

Necropsy was performed twenty-four hours after dosing. Tissue samples were collected and placed in 4% formalin in preparation for histological analysis.

Ovaries were allowed to fix 12 hours in 4% formalin, embedded in paraffin, and sectioned at 3 µm. Step sections were mounted onto microscope slides every 50 µm. 50 µm intervals were chosen based on oocyte diameter (80 µM) such that each follicle would be analyzed without redundancy. Sections were stained with hemotoxylin-eosin. Healthy vs. atretic follicles were scored morphologically by the following criteria: oocyte appearance, alignment of granulosa cell layer, and number of granulosa cell layer. Woodruff *et al*., in Growth Factors and the Ovary, ed., A.N. Hirshfield, pp. 291-295 (New York: Plenum Press, 1989); Osman, J. Reprod. Fertil., 73: 261-270 (1985).

Ovaries treated with 3H-thymidine were fixed analogously to non-radioactive samples and sectioned at 3 µM. Slides were dipped in autoradiographic emulsion, exposed for 2-4 weeks at 4°C, and stained.

Follicular diameter was determined using the Bioquant™ image analysis system. Two measurements were taken at right angles to each other at the level of the granulosa cell bridge to the cumulus and oocyte. Duplication of measurements was avoided by holding the first section image in memory and then juxtaposing it to the subsequent section. Follicles that had been measured in the first section were not used.

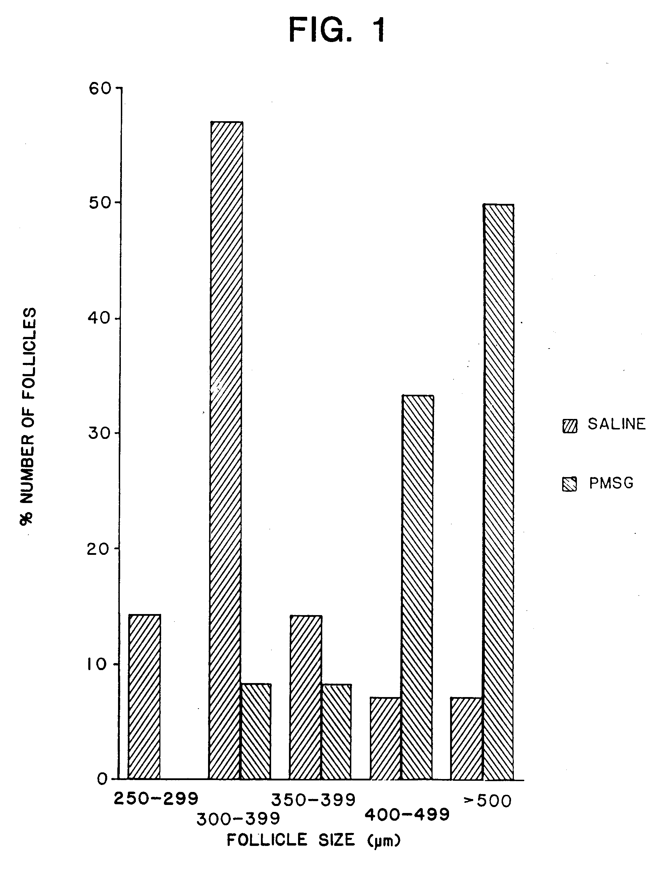

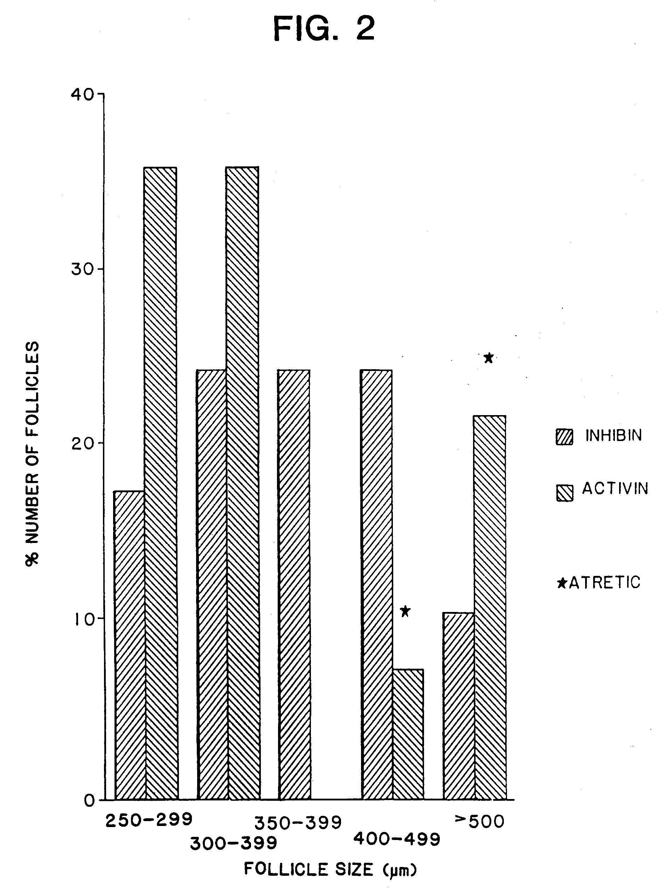

Figure 1 shows the number of follicles in each size class following intrabursal injection of saline or PMSG for a representative set of animals. Figure 2 shows the number of follicles in each size class following intrabursal injection of inhibin A or activin A for a representative set of animals.

Saline injected and untreated (contralateral) ovaries were similar in distribution of follicles. Animals treated with PMSG had follicles distributed from small (250 µm) to large (> 500 µm). Inhibin treatment also caused a wave of follicles moving into larger size classes, although this change was not as pronounced as in PMSG-treated animals. The effect of inhibin on follicular size of PMSG-treated animals was no different from PMSG treatment alone.

Treatment with activin resulted in atretic follicles, as indicated by the asterisks. Interestingly, activin treatment in a PMSG background resulted in a follicular distribution similar to PMSG alone. Follicular size is an indicator of fertility; the size of the follicle increases as it matures and prepares to ovulate and thus is a morphological marker of the maturing oocyte.

Follicular health was also monitored by following incorporation of 3H-thymidine into growing follicles. Saline-treated prepubertal follicles showed low levels of incorporation in mural granulosa cells, theca cells, and cells of the discus proligerus (cells of the cumulus and adjoining cells). Not all follicles in this treatment group were labeled. PMSG treatment resulted in a stimulation of follicular growth with concomitant enhancement of 3H-thymidine incorporation into dividing granulosa and theca cells. Moreover, most follicles of several size classes incorporated label.

Injection of inhibin produced a centripetal pattern of labeled granulosa cells. Two cell layers were labeled, the cells aligning the antral cavity and cells near the basement membrane. When inhibin was coinjected with PMSG, the pattern of labeling was identical to PMSG alone.

Thus, locally administered inhibin influenced folliculogenesis by causing a wave of follicular growth from 250 µm to 350 µm. This size increase is similar to that found in the adult during the estrus to metestrus transition. Moreover, cellular division predominated in the granulosa cells of the antrum and basement layers. This centripetal pattern of mitosis is identical to the pattern reported by Hirshfield for adult cycling animals, Hirshfield, Biol. Reprod., 34: 229-235 (1986). The same pattern was not found in prepubertal animals treated with PMSG. Thus, the addition of inhibin to the immature rat ovary causes a follicular development response identical to adult follicular growth. It appears that the secondary FSH surge is sufficient for follicular selection (adult animal) and follicular development can be maintained by large exogenous doses of PMSG (immature animal), but the production of inhibin is necessary for the maintenance of follicles in the ovulatory pool.

In conclusion, the *in vivo* data demonstrate that inhibin is effective as is FSH in intragonadal upregulating of reproductive function. Therefore, females that do not have fully developed follicles are suitably treated with inhibin to induce ovulation and thereby increase their fertility. Further, inhibin appears to behave somewhat differently *in vivo* from FSH, another fertility drug, indicating that it may be useful in those patients that are non-responsive to FSH.

## Claims (Claims for the following Contracting State(s): AT, BE, CH, DE, DK, FR, GB, IT, LI, LU, NL, SE)

1. Use of inhibin in the manufacture of a medicament for increasing fertility in a female mammal, said medicament being for administration to the ovary of the mammal so that the inhibin acts on the said ovary to influence folliculogenesis thereby to increase fertility.

2. Use according to claim 1 wherein the mammal is human.

3. Use according to claim 1 wherein the medicament is for administration by injection into the ovary.

4. Use according to claim 1 wherein the inhibin is porcine or human inhibin A or B.

5. Use according to claim 4 wherein the inhibin is human inhibin A.

6. Use according to claim 1 wherein the medicament is for use in a method of increasing fertility wherein after inhibin administration, at about midcycle, the oocytes in the follicle of the ovary of the mammal are removed and mixed with spermatozoa, resulting in fertilization.

7. Use according to claim 6 wherein the fertilization takes place in vitro or in vivo.

8. A pharmaceutical product comprising a combined preparation of inhibin and a fertility agent for simultaneous or sequential use in increasing the fertility of a female mammal.

9. A pharmaceutical product according to claim 8 which is a pharmaceutical composition comprising an effective amount of inhibin and an effective amount of a fertility agent in a pharmaceutically acceptable carrier.

10. A pharmaceutical product according to claim 8 or claim 9 wherein the inhibin is porcine or human inhibin A or inhibin B.

11. A pharmaceutical product according to claim 8 or claim 9 wherein the fertility agent is selected from the group consisting of clomiphene citrate, a human menopausal gonadotropin, or a human chorionic gonadotropin.

12. A pharmaceutical product according to claim 8 or claim 9 wherein the fertility agent is follicle stimulating hormone alone or in combination with leutinizing hormone or a gonadotropin releasing hormone antagonist.

13. The pharmaceutical product of claim 12 for administration of a daily amount of follicle stimulating hormone of about 75 to 225/I.U. and a daily amount of gonadotropin releasing hormone antagonist of about 1.0 to 4.0 mg/kg.

14. Use of inhibin and a fertility agent in the manufacture of a medicament of any one of claims 8 to 13.

## Claims (Claims for the following Contracting State(s): GR, ES)

1. Use of inhibin in the manufacture of a medicament for increasing fertility in a female mammal, said medicament being for administration to the ovary of the mammal so that the inhibin acts on the said ovary to influence folliculogenesis thereby to increase fertility.

2. Use according to claim 1 wherein the mammal is human.

3. Use according to claim 1 wherein the medicament is for administration by injection into the ovary.

4. Use according to claim 1 wherein the inhibin is porcine or human inhibin A or B.

5. Use according to claim 4 wherein the inhibin is human inhibin A.

6. Use according to claim 1 wherein the medicament is for use in a method of increasing fertility wherein after inhibin administration, at about midcycle, the oocytes in the follicle of the ovary of the mammal are removed and mixed with spermatozoa, resulting in fertilization.

7. Use according to claim 6 wherein the fertilization takes place in vitro or in vivo.

8. Use of inhibin and a fertility agent in the manufacture of a pharmaceutical product for simultaneous or sequential administration of said inhibin and fertility agent, for use in increasing the fertility of a female mammal.

9. Use according to claim 8 wherein said pharmaceutical product comprises an effective amount of inhibin and an effective amount of a fertility agent in a pharmaceutically acceptable carrier.

10. Use according to claim 8 or claim 9 wherein the inhibin is porcine or human inhibin A or inhibin B.

11. Use according to claim 8 or claim 9 wherein the fertility agent is selected from the group consisting of clomiphene citrate, a human menopausal gonadotropin, or a human chorionic gonadotropin.

12. Use according to claim 8 or claim 9 wherein the fertility agent is follicle stimulating hormone alone or in combination with leutinizing hormone or a gonadotropin releasing hormone antagonist.

13. The use of claim 12 wherein the pharmaceutical product is for administration of a daily amount of follicle stimulating hormone of about 75 to 225/I.U. and a daily amount of gonadotropin releasing hormone antagonist of about 1.0 to 4.0 mg/kg.

## Patentansprüche (Patentansprüche für folgende(n) Vertragsstaat(en): AT, BE, CH, DE, DK, FR, GB, IT, LI, LU, NL, SE)

1. Verwendung von Inhibin bei der Herstellung eines Medikaments zur Erhöhung der Fruchtbarkeit weiblicher Säuger, wobei dieses Medikament zur Verabreichung an den Eierstock des Säugers dient, sodaß das Inhibin auf diesen Eierstock die Follikelgenese beeinflussend wirkt und dadurch die Fruchtbarkeit erhöht.

2. Verwendung nach Anspruch 1, worin der Säuger ein Mensch ist.

3. Verwendung nach Anspruch 1, worin das Medikament zur Verabreichung durch Injektion in den Eierstock dient.

4. Verwendung nach Anspruch 1, worin das Inhibin Schweine- oder menschliches Inhibin A oder B ist.

5. Verwendung nach Anspruch 4, worin das Inhibin menschliches Inhibin A ist.

6. Verwendung nach Anspruch 1, worin das Medikament zur Verwendung bei einem Verfahren zur Erhöhung der Fruchtbarkeit dient, worin nach Inhibinverabreichung, ungefähr in der Mitte des Zyklus, die Oozyten im Follikel des Säugereierstocks entfernt und mit Spermatozoen vermischt werden, was Befruchtung zur Folge hat.

7. Verwendung nach Anspruch 6, worin die Befruchtung in vitro oder in vivo erfolgt.

8. Pharmazeutisches Produkt, umfassend ein kombiniertes Präparat aus Inhibin und einem Fruchtbarkeitsmittel zur gleichzeitigen oder aufeinanderfolgenden Verwendung beim Erhöhen der Fruchtbarkeit eines weiblichen Säugers.

9. Pharmazeutisches Produkt nach Anspruch 8, das eine pharmazeutische Zusammensetzung, umfassend eine wirksame Menge an Inhibin und eine wirksame Menge eines Fruchtbarkeitsmittels in einem pharmazeutisch annehmbaren Träger, ist.

10. Pharmazeutisches Produkt nach Anspruch 8 oder 9, worin das Inhibin Schweine- oder menschliches Inhibin A oder Inhibin B ist.

11. Pharmazeutisches Produkt nach Anspruch 8 oder 9, worin das Fruchtbarkeitsmittel aus der Gruppe, bestehend aus Klomiphenzitrat, einem menschlichen Menopausalgonadotropin oder einem menschlichen chorionischen Gonadotropin, ausgewählt wird.

12. Pharmazeutisches Produkt nach Anspruch 8 oder 9, worin das Fruchtbarkeitsmittel ein follikelstimulierendes Hormon ist, allein oder in Kombination mit leutinisierendem Hormon oder einem Gonadotropin freisetzenden Hormonantagonisten.

13. Pharmazeutisches Produkt nach Anspruch 12 zur Verabreichung einer täglichen Menge an follikelstimulierendem Hormon von ungefähr 75 bis 225/I.E. und einer täglichen Menge an einem Gonadotropin freisetzenden Hormonantagonisten von ungefähr 1,0 bis 4,0 mg/kg.

14. Verwendung von Inhibin und einem Fruchtbarkeitsmittel bei der Herstellung eines Medikaments nach irgendeinem der Ansprüche 8 bis 13.

## Patentansprüche (Patentansprüche für folgende(n) Vertragsstaat(en): GR, ES)

1. Verwendung von Inhibin bei der Herstellung eines Medikaments zur Erhöhung der Fruchtbarkeit weiblicher Säuger, wobei dieses Medikament zur Verabreichung an den Eierstock des Säugers dient, sodaß das Inhibin auf diesen Eierstock die Follikelgenese beeinflussend wirkt und dadurch die Fruchtbarkeit erhöht.

2. Verwendung nach Anspruch 1, worin der Säuger ein Mensch ist.

3. Verwendung nach Anspruch 1, worin das Medikament zur Verabreichung durch Injektion in den Eierstock dient.

4. Verwendung nach Anspruch 1, worin das Inhibin Schweine- oder menschliches Inhibin A oder B ist.

5. Verwendung nach Anspruch 4, worin das Inhibin menschliches Inhibin A ist.

6. Verwendung nach Anspruch 1, worin das Medikament zur Verwendung bei einem Verfahren zur Erhöhung der Fruchtbarkeit dient, worin nach Inhibinverabreichung, ungefähr in der Mitte des Zyklus, die Oozyten im Follikel des Säugereierstocks entfernt und mit Spermatozoen vermischt werden, was Befruchtung zur Folge hat.

7. Verwendung nach Anspruch 6, worin die Befruchtung in vitro oder in vivo erfolgt.

8. Verwendung von Inhibin und einem Fruchtbarkeitsmittel bei der Herstellung eines pharmazeutischen Produkts zur gleichzeitigen oder aufeinanderfolgenden Verabreichung dieses Inhibins und Fruchtbarkeitsmittels zum Gebrauch bei der Erhöhung der Fruchtbarkeit eines weiblichen Säugers.

9. Verwendung nach Anspruch 8, worin das pharmazeutische Produkt eine wirksame Menge an Inhibin und eine wirksame Menge eines Fruchtbarkeitsmittels in einem pharmazeutisch annehmbaren Träger umfaßt.

10. Verwendung nach Anspruch 8 oder 9, worin das Inhibin Schweine- oder menschliches Inhibin A oder Inhibin B ist.

11. Verwendung nach Anspruch 8 oder 9, worin das Fruchtbarkeitsmittel aus der Gruppe, bestehend aus Klomiphenzitrat, einem menschlichen Menopausalgonadotropin oder einem menschlichen chorionischen Gonadotropin, ausgewählt wird.

12. Verwendung nach Anspruch 8 oder 9, worin das Fruchtbarkeitsmittel ein follikelstimulierendes Hormon ist, allein oder in Kombination mit leutinisierendem Hormon oder einem Gonadotropin freisetzenden Hormonantagonisten.

13. Verwendung nach Anspruch 12, worin das pharmazeutische Produkt zur Verabreichung einer täglichen Menge an follikelstimulierendem Hormon von ungefähr 75 bis 225/I.E. und einer täglichen Menge an einem Gonadotropin freisetzenden Hormonantagonisten von ungefähr 1,0 bis 4,0 mg/kg dient.

## Revendications (Revendications pour l'(les) Etat(s) contractant(s) suivant(s): AT, BE, CH, DE, DK, FR, GB, IT, LI, LU, NL, SE)

1. Utilisation d'inhibine dans la production d'un médicament destiné à accroître la fécondité chez un mammifère femelle, ledit médicament étant destiné à être administré à l'ovaire du mammifère de telle sorte que l'inhibine agisse sur ledit ovaire en influençant la folliculogénèse pour accroître ainsi la fécondité.

2. Utilisation suivant la revendication 1, dans laquelle le mammifère est l'homme.

3. Utilisation suivant la revendication 1, dans laquelle le médicament est destiné à être administré par injection dans l'ovaire.

4. Utilisation suivant la revendication 1, dans laquelle l'inhibine est l'inhibine A ou B porcine ou humaine.

5. Utilisation suivant la revendication 4, dans laquelle l'inhibine est l'inhibine A humaine.

6. Utilisation suivant la revendication 1, dans laquelle le médicament est destiné à être utilisé dans un procédé pour accroître la fécondité, dans lequel, après administration d'inhibine, approximativement au milieu du cycle, les ovocytes dans le follicule de l'ovaire du mammifère sont prélevés et mélangés à des spermatozoïdes, avec pour résultat la fécondation.

7. Utilisation suivant la revendication 6, dans laquelle la fécondation s'effectue in vitro ou in vivo.

8. Produit pharmaceutique comprenant une préparation mixte d'inhibine et d'un agent fécondostimulant pour une utilisation simultanée ou séquentielle dans l'accroissement de la fécondité d'un mammifère femelle.

9. Produit pharmaceutique suivant la revendication 8, qui est une composition pharmaceutique comprenant une quantité efficace d'inhibine et une quantité efficace d'un agent fécondostimulant dans un véhicule pharmaceutiquement acceptable.

10. Produit pharmaceutique suivant la revendication 8 ou la revendication 9, dans lequel l'inhibine est l'inhibine A ou l'inhibine B porcine ou humaine.

11. Produit pharmaceutique suivant la revendication 8 ou la revendication 9, dans lequel l'agent fécondostimulant est choisi dans le groupe consistant en le citrate de clomifène, une gonadotrophine de femme ménopausée ou une gonadotrophine chorionique humaine.

12. Produit pharmaceutique suivant la revendication 8 ou la revendication 9, dans lequel l'agent fécondostimulant est la folliculostimuline seule ou en association avec l'hormone lutéinisante ou un antagoniste de gonadolibérine.

13. Produit pharmaceutique suivant la revendication 12 pour l'administration d'une quantité journalière de folliculostimuline d'environ 75 à 225 U.I. et d'une quantité journalière d'antagoniste de gonadolibérine d'environ 1,0 à 4,0 mg/kg.

14. Utilisation d'inhibine et d'un agent fécondostimulant dans la production d'un médicament suivant l'une quelconque des revendications 8 à 13.

## Revendications (Revendications pour l'(les) Etat(s) contractant(s) suivant(s): GR, ES)

1. Utilisation d'inhibine dans la production d'un médicament destiné à accroître la fécondité chez un mammifère femelle, ledit médicament étant destiné à être administré à l'ovaire du mammifère de telle sorte que l'inhibine agisse sur ledit ovaire en influençant la folliculogénèse, pour accroître ainsi la fécondité.

2. Utilisation suivant la revendication 1, dans laquelle le mammifère est l'homme.

3. Utilisation suivant la revendication 1, dans laquelle le médicament est destiné à être administré par injection dans l'ovaire.

4. Utilisation suivant la revendication 1, dans laquelle l'inhibine est l'inhibine A ou B porcine ou humaine.

5. Utilisation suivant la revendication 4, dans laquelle l'inhibine est l'inhibine A humaine.

6. Utilisation suivant la revendication 1, dans laquelle le médicament est destiné à être utilisé dans un procédé pour accroître la fécondité dans lequel, après administration d'inhibine, approximativement au milieu du cycle, les ovocytes dans le follicule de l'ovaire du mammifère sont prélevés et mélangés à des spermatozoïdes, avec pour résultat la fécondation.

7. Utilisation suivant la revendication 6, dans laquelle la fécondation s'effectue in vitro ou in vivo.

8. Utilisation d'inhibine et d'un agent fécondostimulant dans la préparation d'un produit pharmaceutique pour l'administration simultanée ou séquentielle de ladite inhibine et dudit agent fécondostimulant, pour une utilisation dans l'accroissement de la fécondité d'un mammifère femelle.

9. Utilisation suivant la revendication 8, dans laquelle le produit pharmaceutique comprend une quantité efficace d'inhibine et une quantité efficace d'un agent fécondostimulant dans un véhicule pharmaceutiquement acceptable.

10. Utilisation suivant la revendication 8 ou la revendication 9, dans laquelle l'inhibine est l'inhibine A ou l'inhibine B porcine ou humaine.

11. Utilisation suivant la revendication 8 ou la revendication 9, dans laquelle l'agent fécondostimulant est choisi dans le groupe consistant en le citrate de clomifène, une gonadotrophine de femme ménopausée ou une gonadotrophine chorionique humaine.

12. Utilisation suivant la revendication 8 ou la revendication 9, dans laquelle l'agent fécondostimulant est la folliculostimuline seule ou en association avec l'hormone lutéinisante ou un antagoniste de gonadolibérine.

13. Utilisation suivant la revendication 12 dans laquelle le produit pharmaceutique est destiné à l'administration d'une quantité journalière de folliculostimuline d'environ 75 à 225 U.I. et d'une quantité journalière d'antagoniste de gonadolibérine d'environ 1,0 à 4,0 mg/kg.
